(19) Europäisches Patentamt
European Patent Office
Office européen des brevets

(11) Veröffentlichungsnummer: **0 213 378**
**A1**

(12) **EUROPÄISCHE PATENTANMELDUNG**

(21) Anmeldenummer: **86110264.8**

(22) Anmeldetag: **25.07.86**

(51) Int. Cl.[4]: **C 07 D 251/16**
C 07 D 251/42, C 07 D 251/48
C 07 D 251/54, A 01 N 47/36

(30) Priorität: **06.08.85 DE 3528100**

(43) Veröffentlichungstag der Anmeldung:
**11.03.87 Patentblatt 87/11**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**

(72) Erfinder: **Kirsten, Rolf, Dr.**
**Carl-Langhans-Strasse 27**
**D-4019 Monheim(DE)**

(72) Erfinder: **Kluth, Joachim, Dr.**
**Kart-Schumacher-Strasse 9**
**D-4018 Langenfeld(DE)**

(72) Erfinder: **Müller, Klaus-Helmut, Dr.**
**Bockhackstrasse 55**
**D-4000 Düsseldorf 13(DE)**

(72) Erfinder: **Pfister, Theodor, Dr.**
**Lichtenberger Strasse 30**
**D-4019 Monheim(DE)**

(72) Erfinder: **Priesnitz, Uwe, Dr.**
**Severinstrasse 58**
**D-5650 Solingen(DE)**

(72) Erfinder: **Riebel, Hans-Jochem, Dr.**
**In der Beek 92**
**D-5600 Wuppertal 1(DE)**

(72) Erfinder: **Roy, Wolfgang, Dr.**
**Walter-Kolb-Strasse 47**
**D-4018 Langenfeld(DE)**

(72) Erfinder: **Santel, Hans-Joachim, Dr.**
**Grünstrasse 9a**
**D-5090 Leverkusen 1(DE)**

(72) Erfinder: **Schmidt, Robert R., Dr.**
**Im Waldwinkel 110**
**D-5060 Bergisch Gladbach 2(DE)**

(54) **1-(2-Oxyaminosulfonylphenylsulfonyl)-3-triazinyl-harnstoffe.**

(57) Die Erfindung betrifft neue 1-(2-Oxyaminosulfonylphenylsulfonyl) -3-triazinyl-harnstoffe der allgemeinen Formel - (I)

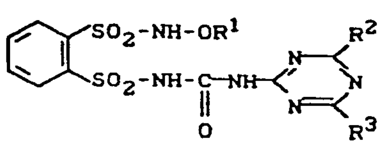

(I)

in welcher

$R^1$ für einen gegebenenfalls substituierten Rest aus der Reihe Alkyl, Alkenyl, Alkinyl, Cycloalkyl, Cyloalkylalkyl, Aralkyl und Aryl steht,

$R^2$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], Amino, für $C_1$-$C_4$-Alkyl- oder Di-($C_1$-$C_4$-alkyl)-amino [welche gegebenenfalls durch Fluor substituiert sind] steht und

$R^3$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/ oder Chlor substituiert ist], für $C_1$-$C_4$-Alkylthio [welches gegebenenfalls durch Fluor und/oder Chlor substituiert ist], Amino, für $C_1$-$C_4$-Alkyl- oder Di-($C_1$-$C_4$-alkyl)-amino [welche gegebenenfalls durch Fluor substituiert sind] steht,

Verfahren zu ihrer Herstellung und ihre Verwendung als Herbizide.

EP 0 213 378 A1

0213378

BAYER AKTIENGESELLSCHAFT
Konzernverwaltung RP
Patentabteilung

5090 Leverkusen, Bayerwerk

Bi/mö/Ke-c
IVb

# 1-(2-Oxyaminosulfonylphenylsulfonyl)-3-triazinyl-harnstoffe

Die Erfindung betrifft neue 1-(2-Oxyaminosulfonylphenylsulfonyl)-3-triazinyl-harnstoffe, Verfahren zu ihrer Herstellung und ihre Verwendung als Herbizide.

Es ist bekannt, daß bestimmte 1-Arylsulfonyl-3-heteroarylharnstoffe, wie z.B. 1-(2-Methoxy-phenylsulfonyl)-3-(4,6-dimethyl-pyrimidin-2-yl)-harnstoff, herbizid wirksam sind. Die Wirkung dieser Verbindungen ist jedoch nicht immer ganz befriedigend (vergl. US-PS 4 169 719).

Es wurden nun neue 1-(2-Oxyaminosulfonylphenylsulfonyl)-3-triazinyl-harnstoffe der allgemeinen Formel (I)

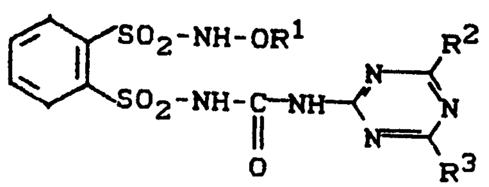

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], Amino, für $C_1$-$C_4$-Alkyl- oder Di-($C_1$-$C_4$-alkyl)-amino [welche gegebenenfalls durch Fluor substituiert sind] steht und

$R^3$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/ oder Chlor substituiert ist], für $C_1$-$C_4$-Alkylthio [welches gegebenenfalls durch Fluor und/oder Chlor substituiert ist], Amino, für $C_1$-$C_4$-Alkyl- oder Di-($C_1$-$C_4$-alkyl)-amino [welche gegebenenfalls durch Fluor substituiert sind] steht,

gefunden.

Man erhält die neuen Verbindungen der Formel (I), wenn man

(a) Benzodisultame der Formel (II)

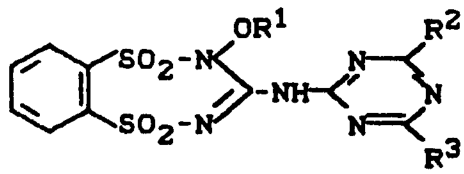

(II)

in welcher

$R^1$, $R^2$ und $R^3$ die oben angegebenen Bedeutungen haben,

mit Wasser gegebenenfalls in Gegenwart von Basen oder Säuren und gegebenenfalls in Gegenwart von Verdünnungsmitteln umsetzt,

oder

(b) Benzol-1,2-disulfonsäure-dichlorid der Formel (III)

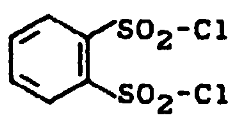

(III)

mit Oxyguanidin-Derivaten der Formel (IV)

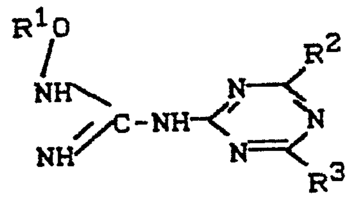

(IV)

in welcher

$R^1$, $R^2$ und $R^3$ die oben angegebene Bedeutung haben,

in Gegenwart von Säureakzeptoren und gegebenenfalls in Gegenwart von Verdünnungsmitteln umsetzt (1. Stufe) und die hierbei erhaltenen Verbindungen der Formel (II) - ohne Zwischenisolierung - mit Wasser gegebenenfalls in Gegenwart von Basen oder Säuren und gegebenenfalls in Gegenwart von Verdünnungsmitteln umsetzt (2. Stufe).

Die neuen 1-(2-Oxyaminosulfonylphenylsulfonyl)-3-triazinyl-harnstoffe der Formel (I) zeichnen sich durch starke herbizide Wirksamkeit aus.

Überraschenderweise zeigen die neuen Verbindungen der Formel (I) erheblich bessere herbizide Wirkung als vorbekannte Harnstoff-Derivate gleicher Wirkungsrichtung.

Gegenstand der Erfindung sind vorzugsweise Verbindungen der Formel (I), in welcher

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkyl-amino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$--Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy--carbonyl substituiert ist], für Benzylhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht, in welcher weiter

$R^2$ für Wasserstoff, Fluor, Chlor, Hydroxy, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$--alkyl)-amino [welche gegebenenfalls durch Fluor substituiert sind] steht und

$R^3$ für Wasserstoff, Fluor, Chlor, Hydroxy, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist], Amino, für $C_1$-$C_3$-Alkyl- oder Di-($C_1$-$C_3$-alkyl)-amino [welche gegebenenfalls durch Fluor substituiert sind] steht.

Gegenstand der Erfindung sind insbesondere Verbindungen der Formel (I), in welcher

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff, Chlor, Methyl, Methoxy, Ethoxy, Methylthio, Ethylthio, Methylamino, Ethylamino, Dimethylamino oder Diethylamino steht und

$R^3$ für Wasserstoff, Chlor, Methyl, Methoxy, Ethoxy, Methylthio, Ethylthio, Methylamino, Ethylamino, Dimethylamino oder Diethylamino steht.

Die bei dem oben unter (a) angegebenen erfindungsgemäßen Herstellungsverfahren ablaufende chemische Reaktion kann beispielsweise durch folgendes Formelschema skizziert werden:

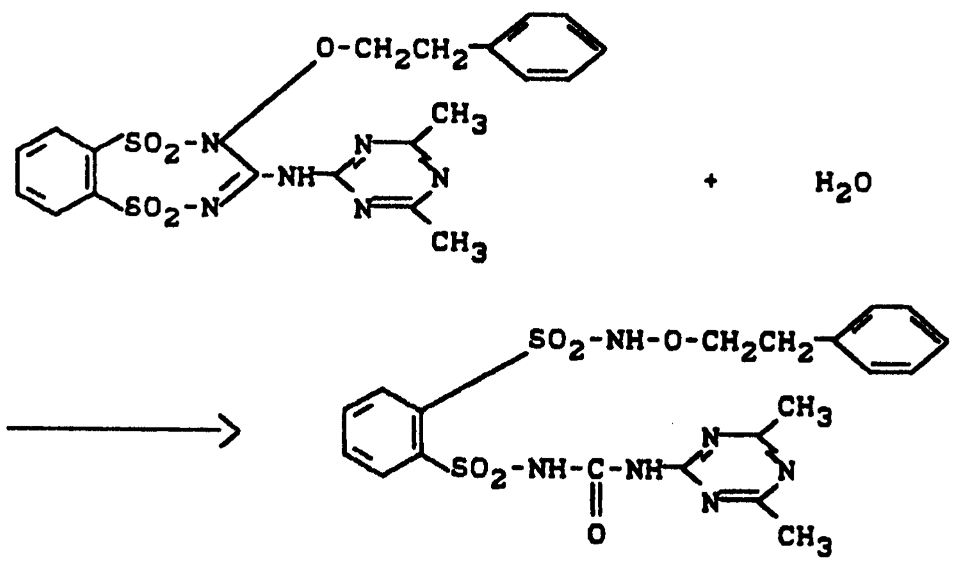


Die bei dem oben unter (b) angegebenen erfindungsgemäßen Herstellungsverfahren ablaufenden Reaktionen können beispielsweise durch folgendes Formelschema skizziert werden:

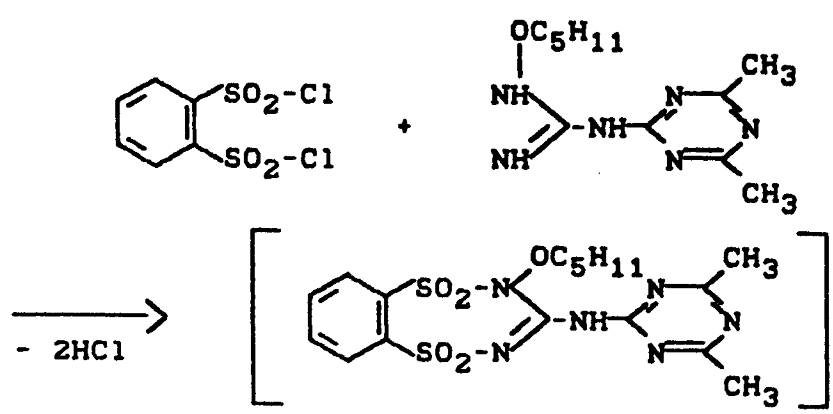

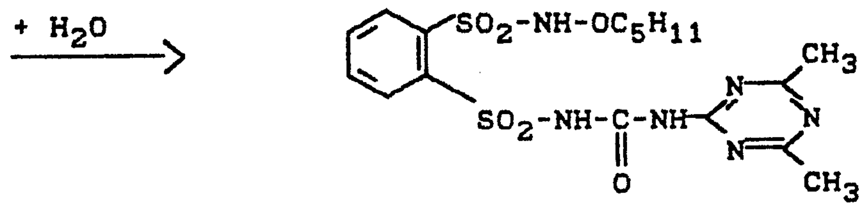


Die beim erfindungsgemäßen Verfahren (a) als Ausgangsstoffe zu verwendenden Benzodisultame sind durch die Formel (II) allgemein definiert.

In Formel (II) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für die Ausgangsstoffe der Formel (II) sind in der nachstehenden Tabelle 1 aufgeführt.

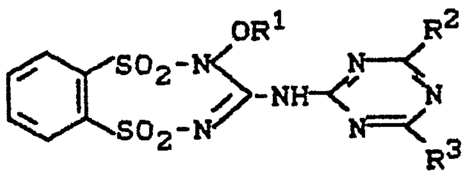
(II)

**Tabelle 1** Beispiele für Ausgangsstoffe der Formel (II)

| $R^1$ | $R^2$ | $R^3$ |
|---|---|---|
| $-CH_3$ | $-OCH_3$ | $-OCH_3$ |
| $-C_2H_5$ | $-OCH_3$ | $-OCH_3$ |
| $-CH_2-CH=CH_2$ | $-OCH_3$ | $-OCH_3$ |
| $-CH_3$ | $-OCH_3$ | $-OC_2H_5$ |
| $-C_4H_9$-n | $-OCH_3$ | $-OC_2H_5$ |
| $-CH_3$ | $-OCH_3$ | $-N(C_2H_5)_2$ |
| $-C_3H_7$-n | $-SCH_3$ | $-NH-C_2H_5$ |
| $-CH_3$ | $-CH_3$ | $-OCH_3$ |
| $-C_8H_{17}$-n | $-CH_3$ | $-OCH_3$ |
| $-CH_2$- 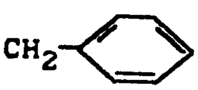 | $-CH_3$ | $-OCH_3$ |
| $-CH_3$ | $-OCH_3$ | $-SCH_3$ |
| $-CH_3$ | $-CH_3$ | $-OC_2H_5$ |
| $-CH_2$- 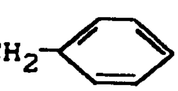 | $-CH_3$ | $-OC_2H_5$ |

Tabelle 1 - Fortsetzung

| $R^1$ | $R^2$ | $R^3$ |
|---|---|---|
| $-CH_3$ | $-SCH_3$ | $-NHC_2H_5$ |
| $-CH_3$ | $-C_2H_5$ | $-OCH_3$ |
| $-CH_3$ | $-CH_3$ | $-CH_3$ |
| $-CH_2-CH=CH_2$ | $-CH_3$ | $-CH_3$ |

Die beim erfindungsgemäßen Verfahren (a) zu verwendenden Verbindungen der Formel (II) sind bisher nicht in der Literatur beschrieben. Man erhält die Verbindungen der Formel (II), wenn man Benzol-1,2-disulfonsäure-dichlorid der Formel (III)

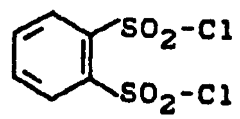

(III)

mit Oxyguanidin-Derivaten der Formel (IV)

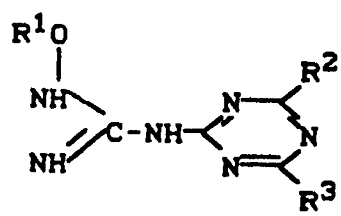

(IV)

in welcher

$R^1$, $R^2$ und $R^3$ die oben angegebenen Bedeutungen haben,

in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid, Chloroform, Tetrahydrofuran oder Dioxan, bei Temperaturen zwischen -30°C und +50°C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende Benzol-1,2-disulfonsäure-dichlorid der Formel (III) ist bereits bekannt (vgl. J. Org. Chem. 31, (1966), 3289-3292).

Die weiter als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (IV) seien beispielsweise genannt:

N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-4,6-(Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N"-methoxy-guanidin, -N"-ethoxy-guanidin, -N"-propoxy-guanidin,-N"-isopropoxy-guanidin, -N"-butoxy--guanidin, -N"-isobutoxy-guanidin, -N"-sec.-butoxy-guanidin, -N"-pentoxy-guanidin, -N"-isopentoxy-guanidin, -N"--hexyloxy-guanidin, -N"-octyloxy-guanidin, -N"-allyloxy--guanidin, -N"-(2-chlor-ethoxy)-guanidin, -N"-(2-fluor--ethoxy)-guanidin, -N"-(2-chlor-propoxy)-guanidin, -N"--(2-fluor-propoxy)-guanidin, -N"-(3-chlor-propoxy)-guanidin, -N"-(4-chlor-butoxy)-guanidin, -N"-methoxycarbonylmethoxy-guanidin, -N"-ethoxycarbonylmethoxy-guanidin, -N"-(1-methoxy-carbonylethoxy)-guanidin, -N"-(1-ethoxycarbonyl-ethoxy)-guanidin, -N"-dimethylamino-carbonyl-methoxy-guanidin, -N"-(2-phenyl-ethoxy)-guanidin, -N"--phenoxy-guanidin, -N"-(4-methylbenzyloxy)-guanidin, -N"--(4-fluorbenzyloxy)-guanidin, -N"-(4-chlor-benzyloxy)--guanidin, -N"-(4-nitro-benzyloxy)-guanidin, -N"-(2,6-dichlor-benzyloxy)-guanidin, -N"-(4-methoxycarbonylbenzyl-oxy)-guanidin und -N"-(4-ethoxycarbonyl-benzyloxy)--guanidin.

Die Ausgangsstoffe der Formel (IV) sind zum Teil bekannt (vergl. EP-A 121 082).

Man erhält die Verbindungen der Formel (IV) wenn man Cyanaminotriazin-Derivate der Formel (V)

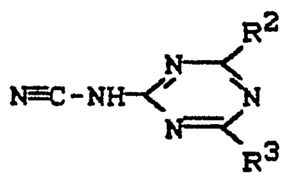

(V)

in welcher

$R^2$ und $R^3$ die oben angegebenen Bedeutungen haben,

mit Hydroxylamin-Derivaten der Formel (VI)

$$H_2N\text{-}OR^1 \qquad (VI)$$

in welcher

$R^1$ die oben angegebene Bedeutung hat,

bzw. deren Hydrochloriden,

gegebenenfalls in Gegenwart von Verdünnungsmitteln, wie z. B. Ethanol, Propanol oder Butanol, bei Temperaturen zwischen -20 °C und 150 °C umsetzt und gegebenenfalls die Umsetzungsprodukte mit Säureakzeptoren, wie z. B. Ammoniak, Kaliumcarbonat oder Natriumhydroxid, behandelt.

Die Cyanaminotriazin-Derivate der Formel (V) sind bekannt (vergl. DE-OS 3 334 455).

Man erhält die Verbindungen der Formel (V) im wesentlichen nach folgenden Synthesewegen:

($a^1$) Durch Umsetzungen von Alkalimetall- oder Erdalkalimetall-Salzen von Cyanamid - wie z.B. Natriumcyanamid oder Calciumcyanamid - mit Chlortriazinen der Formel (VII)

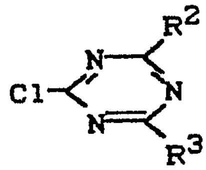

(VII)

in welcher

$R^2$ und $R^3$ die oben angegebenen Bedeutungen haben,

gegebenenfalls in Gegenwart von Säureakzeptoren und gegebenenfalls in Gegenwart von Verdünnungsmitteln, wie z. B. Ethanol, Aceton, Acetonitril, Dimethylformamid, Dimethylsulfoxid oder Wasser, bei Temperaturen zwischen -10 °C und 100 °C. Nach Einengen und Auflösen des Rückstandes in Wasser können die Cyanaminotriazin-Derivate der Formel (V) durch Ansäuern, z. B. mit Salzsäure, ausgefällt und durch Absaugen isoliert werden.

Die als Ausgangsstoffe für das vorausgehend unter ($a^1$) beschriebene Herstellungsverfahren für die Cyanaminotriazin-Derivate der Formel (V) zu verwendenden Chlortriazine der Formel (VII) sind bekannt und/oder können nach an sich bekannten Verfahren hergestellt werden (vgl. US-PS 3 154 547 und US-PS 4 160 037).

In einem weiteren Verfahren erhält man Cyanaminotriazin-Derivate der Formel (V), wenn man

($a^2$) Dichlortriazine der Formel (VIIa)

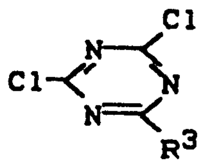

(VIIa)

in welcher

$R^3$ die oben angegebene Bedeutung hat,

mit Alkalimetallsalzen oder Erdalkalimetallsalzen von Cyanamid, wie z.B. mit Dinatriumcyanamid, in Gegenwart eines Verdünnungsmittels, wie z.B. Wasser, bei Temperaturen zwischen -10°C und +50°C umsetzt und das hierbei anfallende Metallsalz des Monochlor-cyanaminotriazins der Formel (Va)

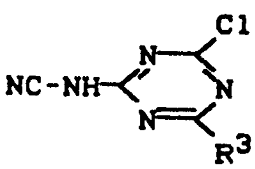

(Va)

in welcher

$R^3$ die oben angegebene Bedeutung hat,

mit einer Säure, wie z.B. Salzsäure, in das freie Monochlor-cyanaminotriazin der Formel (Va) umwandelt.

Aus den Monochlor-cyanaminotriazinen der Formel (Va) können durch Umsetzung mit Alkoholen bzw. Alkanthiolen oder mit Alkalimetallsalzen dieser Verbindungen bzw. mit Monoalkyl- oder Dialkylaminen die entsprechenden Cyanaminotriazine der Formel (V) in welcher $R^2$ für Alkoxy, Alkylthio, Alkylamino oder Dialkylamino steht, hergestellt werden.

Die weiter als Ausgangsstoffe zu verwendenden Hydroxylamin-Derivate der Formel (VI) sind ebenfalls bekannt und können nach an sich bekannten Verfahren hergestellt werden (vergl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Das beim erfindungsgemäßen Verfahren (b) als Ausgangsstoff zu verwendende Benzol-1,2-disulfonsäurechlorid der Formel (III) ist bereits bekannt (vgl. J. Org. Chem. 31, (1966), 3289 - 3292).

Die beim erfindungsgemäßen Verfahren (b) als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Beispiele für Verbindungen der Formel (IV) wurden bereits weiter oben im Zusammenhang mit der Beschreibung der Ausgangsstoffe für Verfahren (a) genannt. Die Herstellung der Ausgangsstoffe der Formel (IV) wurde bereits oben im Zusammenhang mit der Beschreibung der Ausgangsstoffe für Verfahren (a) beschrieben.

Das erfindungsgemäße Verfahren (a) zur Herstellung der neuen Verbindungen der Formel (I) wird vorzugsweise in Wasser als Lösungsmittel durchgeführt. Als weitere Verdünnungsmittel kommen alle inerten organischen Lösungsmittel, vorzugweise jedoch aprotische polare Solventien in Betracht. Hierzu gehören beispielsweise Ketone, wie z.B. Aceton und Methylethylketon, Nitrile, wie z.B. Acetonitril und Propionsäurenitril, Dimethylsulfoxid, Sulfolan, 1,2-Dimethoxyethan und Dioxan.

Verfahren (a) wird bevorzugt in Gegenwart von Basen oder Säuren durchgeführt. Als Basen kommen vorzugsweise Alkalimetall- oder Erdalkalimetall-hydroxide, wie z.B. Natrium-, Kalium- und Calcium-hydroxid, tertiäre Amine, wie z.B. Triethylamin, N,N-Dimethylanilin und N,N-Dimethyl-benzylamin, sowie Stickstoffheterocyclen, wie z.B. Pyridin oder Diazabicyclooctan (DABCO) in Betracht. Als Säuren sind besonders Mineralsäuren geeignet, wie z.B. Chlorwasserstoffsäure, Bromwasserstoffsäure oder Schwefelsäure.

Die Reaktionstemperaturen können beim erfindungsgemäßen Verahren (a) innerhalb eines größeren Bereiches variiert werden. Im allgemeinen arbeitet man zwischen 0°C und

+100°C, vorzugsweise zwischen 10°C und +80°C. Das erfindungsgemäße Verfahren (a) wird im allgemeinen bei Normaldruck durchgeführt.

Zur Durchführung von Verfahren (a) setzt man je Mol Benzodisultam der Formel (II) im allgemeinen zwischen 1 und 100 Mol, vorzugsweise zwischen 5 und 50 Mol Wasser und gegebenenfalls zwischen 1 und 3 Mol, vorzugsweise zwischen 1 und 2 Mol einer Base oder Säure ein.

Die Reaktionskomponenten werden gewöhnlich bei Raumtemperatur zusammengegeben und das Reaktionsgemisch wird bis zum Reaktionsende gerührt.

Die Aufarbeitung kann auf übliche Weise durchgeführt werden; beipsielsweise indem man - z.B. mit Salzsäure - ansäuert, auf etwa die Hälfte des Volumens einengt und das kristallin anfallende Produkt der Formel (I) durch Absaugen isoliert.

Das erfindungsgemäße Verfahren (b) zur Herstellung der neuen Verbindungen der Formel (I) wird vorzugsweise unter Verwendung von Verdünnungsmitteln durchgeführt. Als solche kommen praktisch alle inerten organischen, vorzugsweise aprotisch polare Solventien in Betracht. Hierzu gehören gegebenenfalls substituierte Kohlenwasserstoffe, wie z.B. Methylenchlorid, Chloroform, 1,2-Dichlorethan, Toluol,

Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei der ersten Stufe von Verfahren (b) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen (DBU), Pyridin, 2-Methyl-5-ethyl-pyridin und 4-Dimethylaminopyridin. - Für die bei der zweiten Stufe von Verfahren (b) gegebenenfalls zu verwendenden Basen oder Säuren gelten die entsprechenden, oben für Verfahren (a) gemachten Angaben.

Die Reaktionstemperaturen können bei Verfahren (b) innerhalb eines größeren Bereiches variiert werden. Im allgemeinen arbeitet man zwischen -80°C und + 100°C, vorzugsweise zwischen -30°C und +50°C. Das erfindungsgemäße Verfahren (b) wird im allgemeinen bei Normaldruck durchgeführt.

Zur Durchführung von Verfahren (b) setzt man in der ersten Stufe je Mol Oxyguanidin-Derivat der Formel (IV) im allgemeinen zwischen 1 und 2 Mol, vorzugsweise zwischen 1,0 und 1,2 Mol Benzol-1,2-disulfonsäure-dichlorid der Formel (III) und 2 bis 5 Mol, vorzugsweise 2 bis 3 Mol, eines Säureakzeptors und anschließend zwischen 1 und 100 Mol, vorzugsweise zwischen 5 und 50 Mol Wasser, sowie gegebenenfalls zwischen 1 und 3 Mol, vorzugsweise zwischen 1 und 2 Mol einer Base oder Säure ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung kann auf übliche Weise durchgeführt werden; beispielsweise indem man gegebenenfalls einengt und/oder mit einem mit Wasser praktisch nicht mischbaren organischen Lösungsmittel, wie z.B. Methylenchlorid, verdünnt, mit verdünnter Salzsäure und mit Wasser wäscht, trocknet, filtriert und einengt. Das im Rückstand verbleibende Produkt der Formel (I) wird durch Verreiben mit einem geeigneten organischen Lösungsmittel, wie z.B. Ethanol, zur Kristallisation gebracht und durch Absaugen isoliert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern

erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich zur Bekämpfung mono- und dikotyler Unkräuter in monokotylen Kulturen im Vor- und im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannten Herbizide wie z.B. N-(2- Benzthiazolyl)-N,N'-dimethyl-harnstoff, 3-(3-Chlor-4- methylphenyl)-1,1-dimethylharnstoff, 3-(4-isopropylphenyl)-1,1-dimethylharnstoff, 4-Amino-6-(1,1-dimethyl ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylester, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

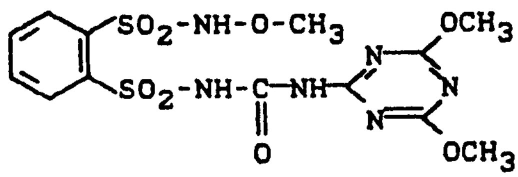

(Verfahren (a))

Eine Mischung von 4,3 g (0,01 Mol) der Verbindung nachstehender Strukturformel

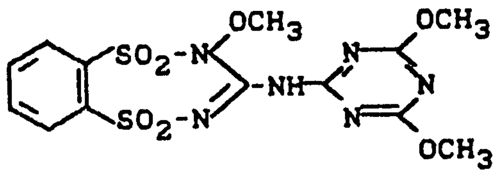

und 40 ml Wasser wird unter heftigem Rühren mit 5 ml konzentrierter Salzsäure versetzt. Man rührt 2 Stunden bei 20 bis 30 °C weiter und saugt dann den entstandenen Niederschlag ab.

Nach dem Trocknen erhält man 3,9 g (87 % der Theorie) 1-(2-Methoxyaminosulfonylphenylsulfonyl)-3-(4,6-dimethoxy-1,3,5-triazin-2-yl)-harnstoff vom Schmelzpunkt 162 °C (Zers.).

Beispiel 2

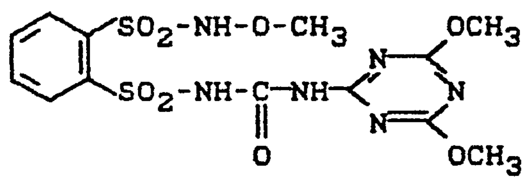

(Verfahren (b))

7,1 g (0,025 Mol) Benzol-1,2-disulfonsäurechlorid werden portionsweise bei -10 °C zu einer Mischung von 5,6 g (0,025 Mol) N'-(4,6-Dimethoxy-1,3,5-triazin-2-yl)-N''-methoxy-guanidin und 50 ml Pyridin gegeben. Man rührt 3 Stunden bei -10 °C und dann 12 Stunden bei 20 - 25 °C nach. Anschließend wird das Lösungsmittel abgezogen und der Rückstand in 200 ml Wasser aufgenommen. Es wird filtriert. Das Filtrat wird mit konzentrierter Salzsäure angesäuert. Nach ca. 1 Stunde wird der entstandene Niederschlag abgesaugt, mit Methanol gewaschen und dann getrocknet.

Man erhält 6,1 g (54 % der Theorie) 1-(2-Methoxyaminosulfonylphenylsulfonyl)-3-(4,6-dimethoxy-1,3,5-triazin-2-yl)-harnstoff vom Schmelzpunkt 160 °C.

Nach den in den vorausgehenden Beispielen 1 und 2 exemplarisch beschriebenen Verfahren können auch die in der nachstehenden Tabelle 2 aufgeführten Verbindungen der Formel (I) hergestellt werden:

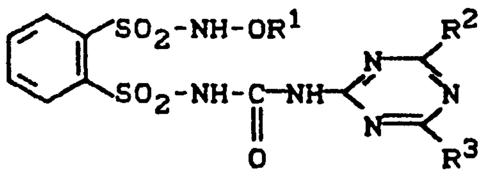

(I)

Tabelle 2

| Beisp.-Nr. | $R^1$ | $R^2$ | $R^3$ | Schmelzpunkt [°C] |
|---|---|---|---|---|
| 3 | $-C_2H_5$ | $-OCH_3$ | $-OCH_3$ | |
| 4 | $-CH_2-CH=CH_2$ | $-OCH_3$ | $-OCH_3$ | |
| 5 | $-CH_3$ | $-OCH_3$ | $-OC_2H_5$ | |
| 6 | $-C_4H_9$ | $-OCH_3$ | $-OC_2H_5$ | |
| 7 | $-CH_3$ | $-OCH_3$ | $-N(C_2H_5)_2$ | |
| 8 | $-C_3H_7$-n | $-SCH_3$ | $-NHC_2H_5$ | |
| 9 | $-CH_3$ | $-CH_3$ | $-OCH_3$ | 174 |
| 10 | $-C_8H_{17}$-n | $-CH_3$ | $-OCH_3$ | |

<u>Tabelle 2</u> - Fortsetzung

| Beisp.-Nr. | $R^1$ | $R^2$ | $R^3$ | Schmelzpunkt [°C] |
|---|---|---|---|---|
| 11 | $-CH_2-$ 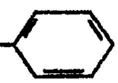 | $-CH_3$ | $-OCH_3$ | 177 |
| 12 | $-CH_3$ | $-OCH_3$ | $-SCH_3$ | |
| 13 | $-CH_3$ | $-CH_3$ | $-OC_2H_5$ | 201 |
| 14 | $-CH_2-$ 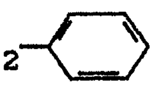 | $-CH_3$ | $-OC_2H_5$ | 212 |
| 15 | $-CH_3$ | $-SCH_3$ | $-NHC_2H_5$ | |
| 16 | $-CH_3$ | $-C_2H_5$ | $-OCH_3$ | |
| 17 | $-CH_3$ | $-CH_3$ | $-CH_3$ | $> 240$ |
| 18 | $-CH_2-CH=CH_2$ | $-CH_3$ | $-CH_3$ | |
| 19 | $-CH_2-CH(CH_3)_2$ | $-CH_3$ | $-OC_2H_5$ | 156 |
| 20 | $-CH_2-CH(CH_3)_2$ | $-CH_3$ | $-OCH_3$ | 176 |
| 21 | $-CH(CH_3)_2$ | $-CH_3$ | $-OC_2H_5$ | 183 |
| 22 | $-CH(CH_3)_2$ | $-OC_2H_5$ | $-NHCH_3$ | 208 |
| 23 | $-CH_3$ | $-OCH_3$ | $-NHCH_3$ | 197 |
| 24 | $-CH_2-$ 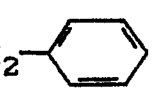 | $-OC_2H_5$ | $-NHCH_3$ | 197 |

Tabelle 2 - Fortsetzung

| Beisp.-Nr. | $R^1$ | $R^2$ | $R^3$ | Schmelzpunkt [°C] |
|---|---|---|---|---|
| 25 | $-CH(CH_3)_2$ | $-CH_3$ | $-OCH_3$ | 192 |
| 26 | $-CH_3$ | $-OC_2H_5$ | $-NHCH_3$ | 215 |
| 27 | $-CH_2$ 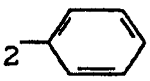 | $-OCH_3$ | $-OCH_3$ | 155 |
| 28 | $-CH_3$ | $-CH_3$ | $-N(CH_3)_2$ | 182 |
| 29 | $-CH_2$ 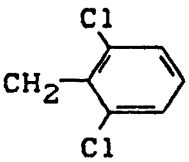 | $-CH_3$ | $-CH_3$ | 154 |
| 30 | $-CH_3$ | $-CH_3$ | $-SCH_3$ | |
| 31 | $-CH_2CH(CH_3)_2$ | $-CH_3$ | $-N(CH_3)_2$ | 170 |
| 32 | $-CH_3$ | $-CH_3$ | $-NHCH_3$ | 202 |
| 33 | $-CH(CH_3)_2$ | $-CH_3$ | $-NHCH_3$ | 267 |
| 34 | $-CH_2CH(CH_3)_2$ | $-CH_3$ | $-NHCH_3$ | |
| 35 | $-CH_3$ | $-CH_3$ | $-NHC_2H_5$ | |
| 36 | $-CH(CH_3)_2$ | $-CH_3$ | $-NHC_2H_5$ | |
| 37 | $-CH_2CH(CH_3)_2$ | $-CH_3$ | $-NHC_2H_5$ | |

Herstellung der Ausgangsverbindungen der Formel (II)

Beispiel (II-1)

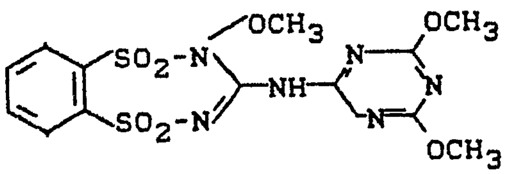

7,1 g (0,025 Mol) Benzol-1,2-disulfonsäurechlorid werden portionsweise bei -10 °C zu einer Mischung von 5,6 g (0,025 Mol) N'-(4,6-Dimethoxy-1,3,5-triazin-2-yl)-N''-methoxy-guanidin, 6 g (0,025 Mol) DABCO und 60 ml Methylenchlorid gegeben. Man rührt 15 Stunden bei 0 bis -5 °C nach. Dann wird das Reaktionsgemisch mit eisgekühlter verdünnter Salzsäure und Eiswasser gewaschen. Die Methylenchloridlösung wird getrocknet und eingeengt.

Nach dem Andestillieren erhält man 4,6 g (43 % der Theorie) der Verbindung der oben angegebenen Strukturformel in Form einer amorphen Masse.

Die Substanz wird durch das $H^1$-NMR-Spektrum charakterisiert.

Nach dem im vorausgehenden Beispiel exemplarisch beschriebenen Verfahren können die in der nachstehenden Tabelle 3 aufgeführten Verbindungen der Formel (II) hergestellt werden:

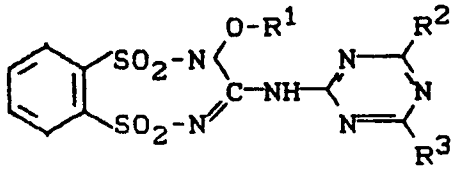

(II)

<u>Tabelle 3</u>

| Beisp.-Nr. | $R^1$ | $R^2$ | $R^3$ | Schmelzpunkt [°C] |
|---|---|---|---|---|
| II-2 | $-C_2H_5$ | $-OCH_3$ | $-OCH_3$ | |
| II-3 | $-CH_2-CH=CH_2$ | $-OCH_3$ | $-OCH_3$ | |
| II-4 | $-CH_3$ | $-OCH_3$ | $-OC_2H_5$ | |
| II-5 | $-C_4H_9$-n | $-OCH_3$ | $-OC_2H_5$ | |
| II-6 | $-CH_3$ | $-OCH_3$ | $-N(C_2H_5)_2$ | |
| II-7 | $-C_3H_7$-n | $-SCH_3$ | $-NHC_2H_5$ | |
| II-8 | $-CH_3$ | $-CH_3$ | $-OCH_3$ | amorph |
| II-9 | $-C_8H_{17}$-n | $-CH_3$ | $-OCH_3$ | |
| II-10 | $-CH_2-$ 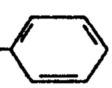 | $-CH_3$ | $-OCH_3$ | |
| II-11 | $-CH_3$ | $-OCH_3$ | $-SCH_3$ | |
| II-12 | $-CH_3$ | $-CH_3$ | $-OC_2H_5$ | |

Tabelle 3 - Fortsetzung

| Beisp.-Nr. | $R^1$ | $R^2$ | $R^3$ | Schmelzpunkt [°C] |
|---|---|---|---|---|
| II-13 | $-CH_2$- 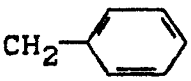 | $-CH_3$ | $-OC_2H_5$ | amorph |
| II-14 | $-CH_3$ | $-SCH_3$ | $-NHC_2H_5$ | |
| II-15 | $-CH_3$ | $-C_2H_5$ | $-OCH_3$ | |
| II-16 | $-CH_3$ | $-CH_3$ | $-CH_3$ | amorph |
| II-17 | $-CH_2-CH=CH_2$ | $-CH_3$ | $-CH_3$ | |
| II-18 | $-CH_2$- 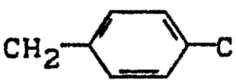 $-COOC_2H_5$ | $-CH_3$ | $-CH_3$ | 195 |
| II-19 | $-CH(CH_3)_2$ | $-CH_3$ | $-OCH_3$ | amorph |
| II-20 | $-CH(CH_3)_2$ | $-CH_3$ | $-OC_2H_5$ | amorph |
| II-21 | $-(CH_2)_7-CH_3$ | $-OCH_3$ | $-OCH_3$ | 110 |
| II-22 | $-CH_2$- 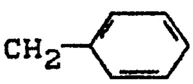 | $-OCH_3$ | $-OCH_3$ | 185 |
| II-23 | $-CH_2$- 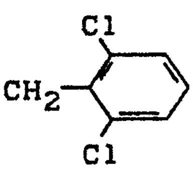 | $-CH_3$ | $-CH_3$ | 150 |

Herstellung von Ausgangsverbindungen der Formel (IV)

Beispiel (IV-1)

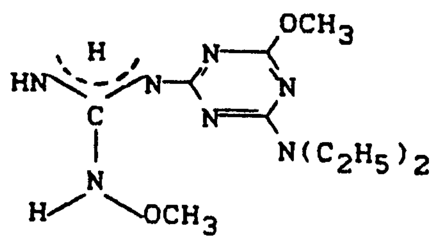

Eine Mischung aus 66,5 g (0,3 Mol) 2-Cyanamino-4-diethylamino-6-methoxy-s-triazin, 50 g (0,8 Mol) O-Methylhydroxylamin-Hydrochlorid und 300 ml Ethanol wird 15 Stunden unter Rückfluß zum Sieden erhitzt. Dann wird filtriert, das Filtrat eingeengt, der Rückstand in ca. 200 ml Wasser aufgenommen, mit Natronlauge alkalisch gestellt und das hierbei kristallin anfallende Produkt durch Absaugen isoliert.

Man erhält 35,0 g (43 % der Theorie) N'-(4-Diethylamino-6-methoxy-s-triazin-2-yl)-N"-methoxy-guanidin vom Schmelzpunkt 112°C.

Analog können die in der nachstehenden Tabelle 4 aufgeführten Verbindungen der Formel (IV) hergestellt werden:

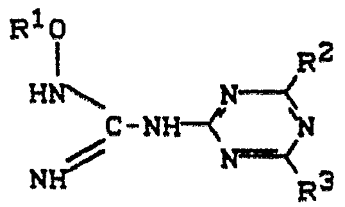

(IV)

Tabelle 4

| Beisp.-Nr. | $R^1$ | $R^2$ | $R^3$ | Schmelzpunkt [°C] |
|---|---|---|---|---|
| IV-2 | $-CH_3$ | $-SCH_3$ | $-NHC_2H_5$ | 117 |
| IV-3 | $-CH_3$ | $-SCH_3$ | $-NHCH_3$ | |
| IV-4 | $-CH_3$ | $-Cl$ | $-N(C_2H_5)_2$ | |
| IV-5 | $-C_2H_5$ | $-OCH_3$ | $-N(C_2H_5)_2$ | |
| IV-6 | $-C_3H_7$-n | $-SCH_3$ | $-NHC_2H_5$ | |
| IV-7 | $-CH(CH_3)_2$ | $-OCH_3$ | $-NHCH_3$ | |
| IV-8 | $-CH_2-CH=CH_2$ | $-OCH_3$ | $-N(CH_3)_2$ | |
| IV-9 | $-C_4H_9$-n | $-OCH_3$ | $-N(CH_3)_2$ | |

Tabelle 4 Fortsetzung

| Beisp.-Nr. | $R^1$ | $R^2$ | $R^3$ | Schmelzpunkt (°C) |
|---|---|---|---|---|
| IV-10 | $-CH_2$ 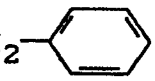 | $-OCH_3$ | $-N(CH_3)_2$ | |
| IV-11 | $-CH_2COOC_2H_5$ | $-OCH_3$ | $-N(CH_3)_2$ | |
| IV-12 | $-CH_2$ 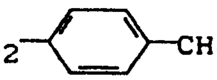 $CH_3$ | $-OCH_3$ | $-N(CH_3)_2$ | |
| IV-13 | $-CH_3$ | $-CH_3$ | $-OCH_3$ | 126 |
| IV-14 | $-CH_2$ 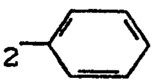 | $-CH_3$ | $-OCH_3$ | 112 |
| IV-15 | $-C_8H_{17}$ | $-CH_3$ | $-OCH_3$ | 95 |
| IV-16 | $-CH_2$ 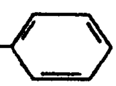 | $-SCH_3$ | $-NHC_2H_5$ | 122 |
| IV-17 | $-CH_3$ | $-CH_3$ | $-OC_2H_5$ | |
| IV-18 | $-CH_2$ 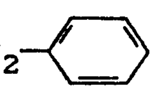 | $-CH_3$ | $-OC_2H_5$ | $n_D^{21}$ = 1.5824 |
| IV-19 | $-CH_3$ | $-CH_3$ | $-CH_3$ | 112 |

Tabelle 4 -Fortsetzung

| Beisp.-Nr. | $R^1$ | $R^2$ | $R^3$ | Schmelzpunkt (°C) |
|---|---|---|---|---|
| IV-20 | $-CH_2-$ 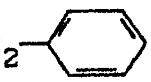 | $-CH_3$ | $-CH_3$ | |
| IV-21 | $-CH_2-CH=CH_2$ | $-CH_3$ | $-CH_3$ | |
| IV-22 | $-CH_3$ | $-OCH_3$ | $-OCH_3$ | 107 |
| IV-23 | $-CH_2-$ 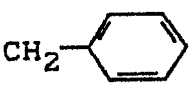 | $-OCH_3$ | $-OCH_3$ | 127 |
| IV-24 | $-CH_2-C(CH_3)_3$ | $-CH_3$ | $-CH_3$ | |
| IV-25 | $-CH_2CH_2-OCH_3$ | $-OCH_3$ | $-OCH_3$ | |
| IV-26 | $-CH_2CH_2-OC_2H_5$ | $-CH_3$ | $-OCH_3$ | |
| IV-27 | $-CH_2CH_2-SCH_3$ | $-CH_3$ | $-OC_2H_5$ | |
| IV-28 | $-CH_2CH_2-SC_2H_5$ | $-CH_3$ | $-CH_3$ | |
| IV-29 | $-CH_2-CONH_2$ | $-OCH_3$ | $-OCH_3$ | |
| IV-30 | $-CH_2-CH=CHCl$ | $-CH_3$ | $-OCH_3$ | |

Tabelle 4 Fortsetzung

| Beisp.-Nr. | $R^1$ | $R^2$ | $R^3$ | Schmelzpunkt ($^\circ C$) |
|---|---|---|---|---|
| IV-31 | -CH 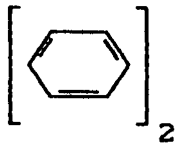 | $-CH_3$ | $-CH_3$ | |
| IV-32 | -C 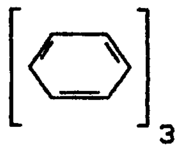 | $-CH_3$ | $-CH_3$ | |
| IV-33 | $-CH_3$ | $-C_2H_5$ | $-OCH_3$ | |
| IV-34 | $-CH_3$ | $-SCH_3$ | $-OCH_3$ | |
| IV-35 | $-CH_3$ | $-OC_2H_5$ | $-OCH_3$ | |
| IV-36 | $-CH_3$ | $-NH-C_2H_5$ | $-OCH_3$ | |
| IV-37 | $-CH_3$ | $-N(CH_3)_2$ | $-OCH_3$ | |
| IV-38 | $-CH_3$ | $-CH_3$ | $-SCH_3$ | |
| IV-39 | $-CH_3$ | $-NH-CH_3$ | $-CH_3$ | |
| IV-40 | $-CH_3$ | $N(CH_3)_2$ | $-CH_3$ | |

Herstellung von Ausgangsstoffen der Formel (V)

Beispiel (V-1)

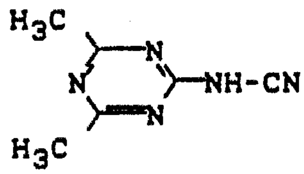

(Verfahren ($a^1$))

Zu einer Suspension von 28,7 g (0,2 Mol) 2-Chlor-4,6-dimethyl-1,3,5-triazin in 100 ml Eiswasser wird unter starkem Rühren in 4 Stunden eine Lösung von 18,0 g (0,21 Mol) Cyanamiddinatriumsalz in 100 ml Wasser bei einer Temperatur von 0 °C bis 5 °C getropft. Anschließend wird bei 20 °C nachgerührt und 15 bis 16 Stunden stehengelassen. Nach Ansäuern mit konz. Salzsäure auf pH = 2 wird abgesaugt, viermal mit 20 ml Eiswasser gewaschen und getrocknet.

Man erhält 19,7 g (66 % der Theorie) 2-Cyanamino-4,6-dimethyl-1,3,5-triazin vom Schmelzpunkt 241 °C (Zers.).

Beispiel (V-2)

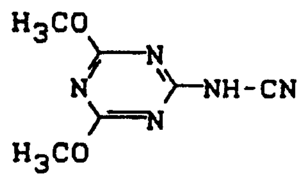

(Verfahren ($a^2$))

Zu einer Suspension von 6 g (0,032 Mol) 4-Chlor-2-cyanamino-6-methoxy-1,3,5-triazin in 50 ml Methanol tropft man bei 20°C bis 30°C eine Lösung von 1,6 g (0,069 Mol) Natrium in 20 ml Methanol. Anschließend wird bei 20°C 15 Stunden nachgerührt. Das Lösungsmittel wird abdestilliert, der Rückstand in 30 ml Wasser gelöst und mit Salzsäure angesäuert. Die entstandenen Kristalle werden abgesaugt und getrocknet.

Man erhält 5,9 g (92 % der Theorie) 2-Cyanamino-4,6-dimethoxy-1,3,5-triazin. Das Produkt wird durch $^1H$-NMR-Spektren charakterisiert.

Analog Beispiel (V-1) und (V-2) können die in der nachstehenden Tabelle 5 aufgeführten Verbindungen der Formel (V) hergestellt werden:

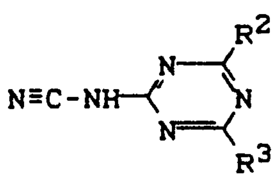

(V)

Tabelle 5

| Beisp.-Nr. | $R^2$ | $R^3$ | Schmelzpunkt (°C) |
|---|---|---|---|
| V-3 | $-OCH_3$ | $-N(C_2H_5)_2$ | 114 |
| V-4 | $-SCH_3$ | $-NHC_2H_5$ | |
| V-5 | $-OCH_3$ | $-NHCH_3$ | 210 |
| V-6 | -Cl | $-N(C_2H_5)_2$ | 156 |
| V-7 | $-OCH_3$ | $-CH_3$ | 184 |
| V-8 | $-OCH(CH_3)_2$ | $-CH_3$ | |
| V-9 | $-SCH_3$ | $-CH_3$ | |
| V-10 | $-SC_2H_5$ | $-CH_3$ | |
| V-11 | $-SCH(CH_3)_2$ | $-CH_3$ | |
| V-12 | $-NHCH_3$ | $-CH_3$ | |
| V-13 | $-NHCH(CH_3)_2$ | $-CH_3$ | |
| V-14 | $-N(CH_3)_2$ | $-CH_3$ | |
| V-15 | $-OC_2H_5$ | $-CH_3$ | 195 (Zers.) |

Tabelle 5 -Fortsetzung

| Beisp.-Nr. | $R^2$ | $R^3$ | Schmelzpunkt (°C) |
|---|---|---|---|
| V-16 | $-C_2H_5$ | $-OCH_3$ | |
| V-17 | $-SCH_3$ | $-OCH_3$ | |
| V-18 | $-OC_2H_5$ | $-OCH_3$ | |
| V-19 | $-OCH_3$ | $-NHCH_3$ | |
| V-20 | $-OCH_3$ | $-N(CH_3)_2$ | |

Herstellung von Ausgangsstoffen der Formel (Va)

Beispiel (Va-1)

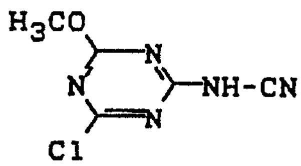

Eine Lösung von 9 g (0,043 Mol) 4-Chlor-2-cyanamino-6-methoxy-1,3,5-triazin-Natriumsalz in 90 ml Wasser wird mit Salzsäure angesäuert und die entstandenen Kristalle werden anschließend abgesaugt.

Man erhält 6,7 g (84 % der Theorie) 4-Chlor-2-cyanamino-6-methoxy-1,3,5-triazin vom Schmelzpunkt >260°C. Das Produkt wird durch $^1$H-NMR-Spektren charakterisiert.

Analog erhält man z. B.:

Beispiel (Va-2)

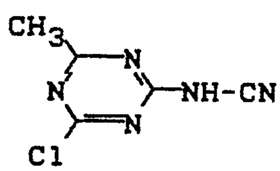

Fp. 105 °C (Zers.)

Beispiel (Va-3)

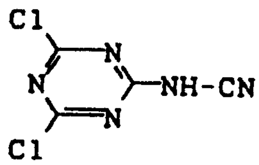

Fp. < 250 °C

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigt z. B. die Verbindung gemäß Herstellungsbeispiel (9) eine bessere Wirkung als die Vergleichsverbindung (A) [= 1-(2-Methoxy-phenylxulonyl)-3-(4,6-dimethyl-pyrimidin-2-yl)-harnstoff].

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigt z. B. die Verbindung gemäß Herstellungsbeispiel (9) eine bessere Wirkung gegen Unkräuter in z. B. Weizen als die Vergleichsverbindung (A).

Patentansprüche

1. 1-(2-Oxyaminosulfonylphenylsulfonyl)-3-triazinyl-harnstoffe der allgemeinen Formel (I)

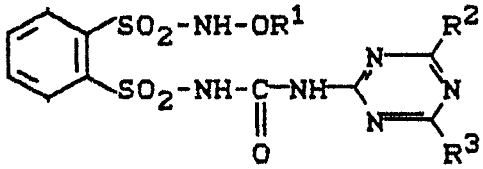

(I)

in welcher

$R^1$ für einen gegebenenfalls substituierten Rest aus der Reihe Alkyl, Alkenyl, Alkinyl, Cycloalkyl, Cylcoalkylalkyl, Aralkyl und Aryl steht,

$R^2$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], Amino, für $C_1$-$C_4$-Alkyl-oder Di-($C_1$-$C_4$-alkyl)-amino [welche gegebenenfalls durch Fluor substituiert sind] steht und

$R^3$ für Wasserstoff, Fluor, Chlor, Brom, Hydroxy, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/ oder Chlor substituiert ist], für $C_1$-$C_4$-Alkylthio [welches gegebenenfalls durch Fluor und/oder Chlor substituiert ist], Amino, für $C_1$-$C_4$-Alkyl- oder Di-($C_1$-$C_4$-alkyl)--amino [welche gegebenenfalls durch Fluor substituiert sind] steht.

2. Verfahren zur Herstellung von 1-(2-Oxyaminosulfonyl-phenylsulfonyl)-3-triazinyl-harnstoffen der allgemeinen Formel (I) gemäß Anspruch 1, dadurch gekennzeichnet, daß man

(a) Benzodisultame der Formel (II)

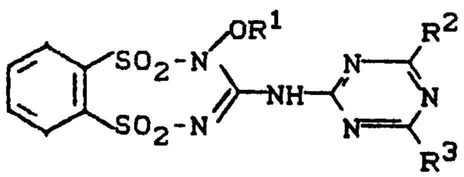

(II)

in welcher

$R^1$, $R^2$ und $R^3$ die in Anspruch 1 angegebenen Bedeutungen haben,

mit Wasser gegebenenfalls in Gegenwart von Basen oder Säuren und gegebenenfalls in Gegenwart von Verdünnungsmitteln umsetzt,
oder

(b) Benzol-1,2-disulfonsäure-dichlorid der Formel (III)

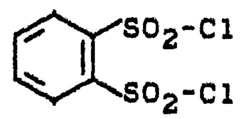

(III)

IL1,5

mit Oxyguanidin-Derivaten der Formel (IV)

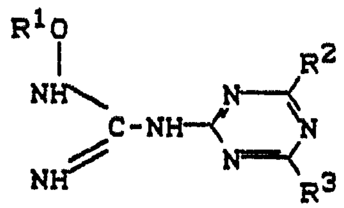

(IV)

in welcher

$R^1$, $R^2$ und $R^3$ die in Anspruch 1 angegebenen Bedeutungen haben,

in Gegenwart von Säureakzeptoren und gegebenenfalls in Gegenwart von Verdünnungsmitteln umsetzt (1. Stufe)

und die hierbei erhaltenen Verbindungen der Formel (II) - ohne Zwischenisolierung - mit Wasser gegebenenfalls in Gegenwart von Basen oder Säuren und gegebenenfalls in Gegenwart von Verdünnungsmitteln umsetzt (2. Stufe).

3. Herbizide Mittel, gekennzeichnet durch einen Gehalt an mindestens einem 1-(2-Oxyaminosulfonylphenylsulfonyl)-3-triazinyl-harnstoff der allgemeinen Formel (I) gemäß Anspruch 1.

4. Verwendung von 1-(2-Oxyaminosulfonylphenylsulfonyl)-3-triazinyl-harnstoffen der allgemeinen Formel (I) gemäß Anspruch 1 zur Bekämpfung von unerwünschtem Pflanzenwachstum.

5. Verfahren zur Herstellung von herbiziden Mitteln, dadurch gekennzeichnet, daß man 1-(2-Oxyaminosulfonylphenylsulfonyl)-3-triazinyl-harnstoffe der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

0213378
Nummer der Anmeldung

EP 86110264.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A2 - 0 124 295 (DU PONT)<br>* Seite 2, Zeile 35; Ansprüche 1,22,23 *<br>-- | 1,3-5 | C 07 D 251/16<br>C 07 D 251/42<br>C 07 D 251/48<br>C 07 D 251/54<br>A 01 N 47/36 |
| A | EP - A1 - 0 084 224 (DU PONT)<br>* Seite 210, Zeile 12; Ansprüche 1,16,17 *<br>-- | 1,3-5 | |
| A,P | US - A - 4 548 638 (HAGEMANN)<br>* Spalte 11, Zeile 5; Ansprüche 1,7,9 *<br>---- | 1,3-5 | |

RECHERCHIERTE SACHGEBIETE (Int. Cl.4)

C 07 D 251/00

Der vorliegende Recherchenbericht wurde für alle Patentansprüche erstellt.

| Recherchenort | Abschlußdatum der Recherche | Prüfer |
|---|---|---|
| WIEN | 24-10-1986 | HAMMER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82